# EUROPEAN PATENT SPECIFICATION

(11) **EP 4 021 417 B1**
(45) Date of publication and mention of the grant of the patent: **04.10.2023**
(21) Application number: 20758240.4
(22) Date of filing: 26.08.2020
(51) Int. Cl.: A61K 9/16, A61K 9/20, A61K 31/192, A61K 31/4415, A61K 31/51, A61K 31/714, A61P 29/00

(54) **SOLID ORAL DOSAGE FORM COMPRISING NAPROXEN AND VITAMIN B6**
FESTE ORALE DOSIERUNGSFORM MIT NAPROXEN UND VITAMIN B6
FORME POSOLOGIQUE ORALE SOLIDE COMPRENANT DU NAPROXÈNE ET DE LA VITAMINE B6

(30) Priority: 26.08.2019 EP 19193616
(43) Date of publication of application: 06.07.2022
(73) Proprietor: DSM IP Assets B.V., 6411 TE Heerlen (NL)
(72) Inventor: KARPUKHIN, Denis, 4303 Kaiseraugst (CH); MA, Zhenbo, 4303 Kaiseraugst (CH); SALMON, Conroy Clive, 4303 Kaiseraugst (CH)
(74) Representative: Sethmann, Svenja
(86) International application number: PCT/EP2020/073783
(87) International publication number: WO 2021/037871

(56) References cited:
- WO-A1-2008/120765
- CN-A- 109 316 458
- US-A1- 2015 132 410
- US-A1- 2015 157 585

## Description

### Technical field

The present invention relates to the use of naproxen for the treatment of pain.

### Background of the invention

As one of the world's most common ailments, low back pain is the leading cause of activity limitation and work absence, imposing a high economic burden on individuals, families, communities, industry, and governments.

The lower back includes the five vertebrae (referred to as L1-L5) in the lumbar region. The spaces between the vertebrae are maintained by round, rubbery pads called intervertebral discs that act like shock absorbers throughout the spinal column to cushion the bones as the body moves. Bands of tissue known as ligaments hold the vertebrae in place, and tendons attach the muscles to the spinal column. Thirty-one pairs of nerves are rooted to the spinal cord and they control body movements and transmit signals from the body to the brain (cf. "Low Back Pain Fact Sheet", National Institute of Neurological Disorders and Stroke, NIH Publication No. 15-5161, 2014).

Currently established global clinical practice guidelines consistently recommend the use of acetaminophen as first-line treatment. As second-line treatment for low back pain, the administration of naproxen is a noteworthy option.

While both acetaminophen and naproxen have well-established efficacy and safety profiles, there exists a significant unmet need for patients whose pain remains uncontrolled despite maximal therapy.

Recent research has highlighted the role of B-vitamins in addressing low back pain and improving global clinical outcomes. Vitamins of the vitamin B complex are commercially available at DSM^{®} Nutritional Products, Switzerland.

While the exact mechanisms for vitamin B complex efficacy in the treatment of low back pain are still largely unknown, the prevailing hypothesis involves increasing afferent inhibitory control of nociceptive neurons at the spinal cord, improving sensory nerve conduction velocity and reducing neuronal hyperexcitability by altering sodium currents in injured dorsal root ganglia (Q. Fu et al. B Vitamins Suppress Spinal Dorsal Horn Nociceptive Neurons in the Cat. Neurosci Lett 1988;95:192-197; C. Jolivalt et al. B Vitamins Alleviate Indices of Neuropathic Pain in Diabetic Rats. Eur J Pharmacol 2009;612:41-47; X. Song et al. Thiamine Suppresses Thermal Hyperalgesia Inhibits Hyperexcitability, and Lessens Alterations of Sodium Currents in Injured Dorsal Root Ganglion Neurons in Rats. Anesthesiology 2009; 110:387-400). Both, naproxen and vitamins of the vitamin B complex are commercially available. To improve patient compliance, there is a need for a fixed-dose combination (FDC) of naproxen and at least one vitamin of the vitamin B complex. The FDC should be a solid oral dosage form which treats low back pain more effectively than naproxen only, which improves patient compliance, which has no or little side effects, is storage stable, is easy to swallow, is easy to manufacture and/or meets quality standards of the pharmaceutical industry. FDC of naproxen and one of vitamin B complexes is known e.g. from US2015/157585.

### Summary of the invention

Compressing naproxen as such into a tablet is very difficult or even impossible. Therefore, naproxen is granulated before compressing it into a tablet.

The easiest manner to provide a fixed-dose combination (FDC) of naproxen and vitamin B6 would be to granulate water-soluble naproxen sodium together with water-soluble vitamin B6, using water as processing solvent for wet granulation. The thus obtained granules could then be compressed into tablets. Aqueous wet-granulation is more cost effective and more sustainable than using an organic solvent as processing solvent. In addition, there is no explosion risk when using water instead of an organic solvent.

Nevertheless, the inventors have chosen a different approach.

The present invention relates to a method of preparing a solid oral dosage form, said method comprising the extragranular addition of vitamin B6 to an intragranular composition, wherein said intragranular composition comprises naproxen or a pharmaceutically acceptable salt thereof. When following this approach, a solid oral dosage form can be manufactured that has the required hardness, friability and/or content uniformity such that it meets quality standards of the pharmaceutical industry.

When applying the method of the invention, a blend is obtained that comprises a) granules and b) vitamin B6, wherein said granules comprise naproxen or a pharmaceutically acceptable salt thereof and at least one binder. Said blend can then be filled into capsules or containers or can be compressed into tablets.

When compressing tablets, capping might occur. The term capping is used when the upper or the lower part of the tablet separates horizontally either partially away from the main body or completely to form a cap when ejected from the press or during the handling process. There are various approaches how capping can be avoided and reduced such as modification of the tooling, adaptation of the press setup, improving weight control or change of powder characteristics.

Surprisingly, in case of a fixed-dose combination (FDC) of naproxen and vitamin B6, capping can be avoided or reduced when naproxen sodium and vitamin B6 are not granulated together.

The fixed-dose combination (FDC) of the invention treats low back pain more effectively than naproxen only and/or improves patient compliance. Therefore, the present invention also relates to the herein described solid oral dosage for use in the treatment of low back pain.

### Detailed description of the invention

### Definitions

The present invention relates to naproxen or a pharmaceutically acceptable salt thereof (e.g. a salt of an alkali metal). The preferred embodiment of the present invention relates to naproxen sodium. Naproxen sodium is a white to creamy white, crystalline powder that is soluble in water. Commercially available naproxen tablets such as Aleve^{®} and Naprosyn^{®} comprise naproxen sodium.

Direct compression is the shortest, most effective and least complex way to produce tablets. The manufacturer can compress a blend of the drug with excipients. No additional processing steps are required. Unfortunately, some powders are difficult to compress even if a readily compactable binder/adhesive is included in the blend, but granules of the same powders are often more easily compressed.

"**Granules**" are formed by granulation. Granulation is the process of forming of granules and involves agglomeration of fine particles into larger granules. Wet granulation and dry granulation are two types of granulation technologies. The granules of the present invention are preferably formed by wet granulation. Wet granulation requires a processing solvent. In the context of the present invention, water is the preferred processing solvent. For agglomeration of fine particles into larger granules, a binder might be necessary. When doing wet granulation, the binder must be adapted to the chosen processing solvent. The granules of the present invention are preferably formed by wet granulation, wherein water is used as processing solvent and wherein a binder suitable for aqueous wet granulation is used. Typically, such binders are soluble in water. Preferred binders are PVP (polyvinylpyrrolidone, also commonly called polyvidone or povidone) and HPMC (hydroxypropyl methylcellulose; short: hypromellose).

When manufacturing a solid pharmaceutical dosage form, a particular compound may be blended with other ingredients prior to granulation and is thus incorporated within the obtained granules. This is referred to as **"intragranular addition".** Alternatively, a particular compound (or a mixture of compounds) may be mixed with already existing, prefabricated granules. This is referred to **"extragranular addition".** After extragranular addition, the obtained blend can then be further processed (e.g. can be compressed into tablets or can be filled into sachets). Thus, an **"extragranular"** compound is not part of a granule (i.e. is outside of a granule) whereas an **"intragranular"** compound is part of a granule. An **"intragranular composition"** is a composition obtained by a granulation process. An example of an intragranular composition are the herein described granules.

The present invention relates to a fixed-dose combination (FDC) comprising at least one B-vitamin. In the context of the present invention, the terms **"B-vitamin"** and **"vitamin of the B complex"** are used interchangeably and refer preferably to vitamin B1, vitamin B6 and vitamin B12.

**"Vitamers"** of a particular vitamin are chemical compounds that relieve a particular vitamin deficiency in a vitamin-deficient biological system. Thus, they are compounds that posses a given vitamin activity. Often, vitamers of a particular vitamin have a similar molecular structure.

In the context of the present invention, the term **"vitamin B1"** refers to any vitamer of vitamin B1. Thus, the term "vitamin B1" includes thiamine, phosphorylated derivatives of thiamine and synthetic derivatives of thiamine such as benfotiamine. Typically, a derivative is a compound that is derived from a similar compound by a chemical reaction. In a preferred embodiment of the invention, the term "vitamin B1" refers thiamine hydrochloride, thiamine mononitrate, benfotiamine or a mixture thereof. The most preferred vitamin B1 is thiamine hydrochloride.

In the context of the present invention, the term **"vitamin B6"** refers to any vitamer of vitamin B6. Thus, the term "vitamin B6" includes pyridoxine, salts of pyridoxine and derivatives of pyridoxine. In a preferred embodiment of the invention, the term "vitamin B6" refers to a salt of pyridoxine. Most preferably, the term "vitamin B6" refers to pyridoxine hydrochloride.

Vitamin B12 is a well-known water-soluble vitamin. In the context of the present invention, the term **"vitamin B12"** refers to any vitamer of vitamin B12 and includes vitamin B12 derivatives and/or metabolites of vitamin B12.

Preferably, however, the term "vitamin B12" refers to cyanocobalamin. Cyanocobalamin may be produced by fermentation using suitable microorganisms. "Crystalline vitamin B12" comprises at least 98 weight-% vitamin B12, based on the total weight of the crystals. Preferably, the blend of the present invention does not comprise any crystalline vitamin B12. A preferred embodiment of the invention relates to a blend comprising a spray-dried formulation of vitamin B12. The expression **"spray-dried formulation of vitamin B12"** refers to a powder which is obtainable by spray drying of an aqueous solution that comprises vitamin B12 and at least one excipient, wherein said at least one excipient is preferably selected from the group consisting of sodium citrate, trisodium citrate, citric acid, maltodextrin and modified food starch. In a preferred embodiment of the invention, the expression "spray-dried formulation of vitamin B12" refers to a powder which is obtainable by spray drying an aqueous solution which comprises cyanocobalamin and at least one excipient, wherein said at least one excipient is preferably selected from the group consisting of sodium citrate, trisodium citrate, citric acid, maltodextrin and modified food starch.

Due to the presence of at least one excipient, the spray-dried formulation of vitamin B12 comprises less than 90 weight-% of vitamin B12, based on the total weight of the spray-dried formulation. The exact concentration of vitamin B12 in the spray-dried formulation of vitamin B12 depends on the amount of excipient in the spray-dried formulation. Preferably, the spray-dried formulation of vitamin B12 of the invention comprises 1 weight-% or less of vitamin B12, based on the total weight of the spray-dried formulation. The person skilled in the art understands that spray-dried formulations of vitamin B12 being free of vitamin B12 are excluded. Thus, the spray-dried formulation of vitamin B12 comprises preferably from 0.01 to 1 weight-%, more preferably from 0.05 to 0.5 weight-% and most preferably 0.1 weight-% vitamin B12, based on the total weight of the spray-dried formulation of vitamin B12. Also preferably, the spray-dried formulation of vitamin B12 as used in the context of the present invention is a water-soluble or water-dispersible powder comprising 1 weight-% or less of cyanocobalamin, based on the total weight of the powder. The person skilled in the art understands that powders being free of vitamin B12 are excluded. Thus, the spray-dried formulation of vitamin B12 is preferably a water-soluble or water-dispersible powder that comprises from 0.01 to 1 weight-%, more preferably from 0.05 to 0.5 weight-% and most preferably 0.1 weight-% cyanocobalamin, based on the total weight of the powder. In the most preferred embodiment of the invention, the expression "spray-dried formulation of vitamin B12" refers to a powder which is obtainable by spray drying an aqueous solution which comprises cyanocobalamin and at least one excipient, wherein said excipient is preferably selected from the group consisting of sodium citrate, trisodium citrate, citric acid, maltodextrin and modified food starch, and wherein said powder comprises 1 weight-% or less of cyanocobalamin, based on the total weight of the powder. Again, the person skilled in the art understands that powders being free of vitamin B12 are excluded.

**"Estimated Average Requirement (EAR)"** is a nutrient intake value that is estimated to meet the requirement of half the healthy individuals in a life stage and gender group. **"Recommended Dietary Allowances (RDA)"** is the dietary intake level that is sufficient to meet the nutrient requirement of nearly all (97 to 98 percent) healthy individuals in a particular life stage and gender group. RDA = EAR + 2 SD_{EAR} or if insufficient data to calculate SD (standard deviation), a factor of 1.2 is used to calculate RDA; RDA = 1.2*EAR. **Adequate Intake (Al)"** is the recommended intake value based on observed or experimentally determined approximations or estimates of nutrient intake by a group (or groups) of healthy people that are assumed to be adequate - used when an RDA cannot be determined. **"Tolerable Upper Intake Level (UL)"** is the highest level of nutrient intake that is likely to pose no risk of adverse health effects for almost all individuals in the general population.

Some laws require that a product comprising a vitamin must contain, at minimum, 100% of the label claimed amount of the vitamin ("**label claim**")**.** To ensure that the content of vitamin in the product meets the requirement of 100% of the label claimed amount throughout the shelf life of the product, manufacturers typically formulate products to contain vitamins in amounts greater than the label claimed amount (i.e., overage amounts or overages) to compensate for loss due to degradation of the vitamin during the product's shelf life, and to compensate for the inherent variability of the manufacturing process and product testing. Typically, this does not apply to active pharmaceutical ingredients (API). For APIs, the label claim corresponds to the actual amount (i.e. no overages).

Solid oral dosage forms are the most important dosage forms for pharmaceuticals and include tablets, capsules, gelcaps, semi-solid gels, powders, sachets, stick-packs, ready-to-mix powders, dry powder inhalers and chewables. In a preferred embodiment of the invention, the term "solid **oral dosage form**" refers to a tablet, a capsule or to a powder. Preferably, the powder is a water-soluble or a water-dispersible powder. The powder of the present invention is preferably enclosed in a container such as a bag, sachet, bottle or stick-pack.

### Solid oral dosage form of the invention

The solid oral dosage form of the invention is a pharmaceutical dosage form which is orally administered to a patient that is in need of naproxen. In case that the solid oral dosage form of the invention is water-soluble or water-dispersible powder, an aqueous solution or dispersion comprising said powder is orally administered to a patient that is in need of naproxen.

The solid oral dosage form of the invention is a fixed-dose combination (FDC) comprising naproxen or a pharmaceutically acceptable salt thereof, vitamin B6 and optionally at least one additional B-vitamin. In a preferred embodiment of the invention, the solid oral dosage form comprises (in addition to vitamin B6 and naproxen or a pharmaceutically acceptable salt thereof) also vitamin B1 and/or vitamin B12. Most preferably, the solid oral dosage form of the present invention comprises naproxen sodium, vitamin B6, vitamin B1 and vitamin B12.

The amount of vitamin B1, vitamin B12 and vitamin B6 in the solid oral dosage form of the invention is guided by the Recommended Dietary Allowances (RDA) of the respective B-vitamin, as published in: Institute of Medicine. 1998. Dietary Reference Intakes for Thiamin, Riboflavin, Niacin, Vitamin B6, Folate, Vitamin B12, Pantothenic Acid, Biotin, and Choline. Washington, DC: The National Academies Press. https://doi.org/10.17226/6015.

In one embodiment of the invention, the solid oral dosage form of the invention comprises preferably of 50-120%, more preferably 50-100% and most preferably 80-100% of the RDA for vitamin B6. A patient may take up to 3 naproxen tablets (e.g. Aleve^{®}) in 24 hours. The inventors suggest replacing these 3 naproxen tablets with 3 fixed-dose combination dosage forms, each comprising approx. 1/3 RDA of the respective B-vitamin. Thus, in a preferred embodiment of the invention, the solid oral dosage form of the invention comprises preferably of 20-50%, more preferably 20-35% and most preferably 25-33.3% of the RDA for vitamin B6. In one embodiment of the invention, the solid oral dosage form of the invention comprises naproxen sodium and vitamin B6, with 200 mg label claim of vitamin B6.

This applies mutatis mutandis also to vitamin B1 and vitamin B12. In one embodiment of the invention, the solid oral dosage form of the invention comprises preferably of 50-120%, more preferably 50-100% and most preferably 80-100% of the RDA for vitamin B1 and/or comprises preferably of 50-120%, more preferably 50-100% and most preferably 80-100% of the RDA for vitamin B12. In a preferred embodiment of the invention, the solid oral dosage form of the invention comprises preferably of 20-50%, more preferably 20-35% and most preferably 25-33.3% of the RDA for vitamin B1 and/or comprises preferably of 20-50%, more preferably 20-35% and most preferably 25-33.3% of the RDA for vitamin B12.

The preferred source of vitamin B12 is cyanocobalamin. Spray-dried formulations of cyanocobalamin are available in pharma-grade at DSM^{®} Nutritional Products, Switzerland. In one embodiment of the invention, the solid oral dosage form of the present invention comprises naproxen sodium and 10 µg-1000 µg, preferably 100 µg-500 µg and most preferably from 180 µg-300 µg vitamin B12. In this embodiment, the solid oral dosage form of the invention is preferably a compressed tablet.

The preferred source of vitamin B6 is pyridoxine hydrochloride. Pharma-grade pyridoxine hydrochloride is available at DSM^{®} Nutritional Products, Switzerland. The level of vitamin B6 may range from 0.5 mg to 300 mg, corresponding to a level of pyridoxine hydrochloride from 0.67 mg to 401.12 mg per serving. In one embodiment of the invention, the solid oral dosage form of the present invention comprises naproxen sodium and 100 mg-400 mg, preferably 150 mg-300 mg and most preferably 200 mg-270 mg pyridoxine hydrochloride. In this embodiment, the solid oral dosage form of the invention is preferably a compressed tablet.

In an also preferred embodiment of the invention, the solid oral dosage form of the invention is a water-soluble or water-dispersible powder which comprises preferably at least one taste-masking agent such as a sweetener (e.g. sucralose), an acid (e.g. citric acid or malic acid) and/or a flavor (e.g. lemon favor or raspberry flavor). Thus, one embodiment of the invention relates to a powder comprising naproxen sodium and from 0.67 mg to 401.12 mg pyridoxine hydrochloride and preferably at least one taste-masking agent.

In an also preferred embodiment of the invention, the solid oral dosage form of the invention is a capsule, wherein each capsule comprises preferably 110 mg naproxen sodium. The serving size is 2 capsules, adding up to 220 mg naproxen sodium (label claim). Thus, one embodiment of the invention relates to a capsule comprising from 100 to 120 mg naproxen sodium and 10 mg-250 mg, preferably 75 mg-150 mg and most preferably 100 mg-135 mg pyridoxine hydrochloride.

In one embodiment of the invention, the solid oral dosage form of the present invention comprises preferably naproxen sodium (220 mg label claim of naproxen sodium or 200 mg label claim of naproxen), vitamin B1 (label claim 100 mg), vitamin B6 (label claim 200 mg), and vitamin B12 (label claim 200 µg). For vitamins, overages above label claim are acceptable or even recommended. For some vitamins, overages above label claim may even be essential.

Some vitamins (and in particular vitamin B12) are light sensitive. Therefore, it is recommended to protect the solid oral dosage form of the invention from light. In one embodiment, the tablet of the invention is coated with a coating that provides for light protection such as Opadry^{®} coatings (commercially available at Colorcon). A preferred embodiment of the invention relates to a coated tablet comprising naproxen sodium, vitamin B6, vitamin B1 and vitamin B12, wherein the coating of the tablet comprises at least one light-protecting agent being preferably titanium dioxide.

This applies mutatis mutandis to the capsule of the invention. In one embodiment, the capsule of the invention comprises a capsule shell that provides for light protection. Capsule shells having customized color are available at Capsugel^{®}.

In addition or alternatively, a light-protecting packaging material may be used. Examples of packaging material that provides for light protection are aluminium and high density polyethylene (HDPE). Thus, one embodiment relates to high density polyethylene (HDPE) bottles comprising the solid oral dosage form of the invention. Another embodiment relates to a blister, wherein said blister provides for light protection and wherein said blister encloses the tablets or capsules of the present invention.

Most sachets and stick packs also provide for light protection. This applies in particular to sachets, bags and stick packs that comprise at least one layer of aluminium. Thus, one embodiment relates to a sachet, bag or stick pack enclosing the solid oral dosage form of the invention, wherein said sachet, bag or stick pack protects the enclosed solid oral dosage form from light, and wherein the solid oral dosage form is a preferably water-soluble or water-dispersible powder.

The present invention also relates to a method of treatment, wherein the solid oral dosage form of the invention is administered to a patient who is in need of naproxen sodium such as a patient suffering from pain. A preferred embodiment of the present invention relates to a method of treatment, wherein the solid oral dosage form of the invention is administered to a patient suffering from pain from arthritis pain, headache, muscular aches, toothache, backache, low back pain and/or menstrual cramps. Most preferably, the present invention relates to a method of treatment, wherein the solid oral dosage form of the invention is administered to a patient suffering from low back pain.

Another embodiment relates to the solid oral dosage form of the invention for use as a medicament. A preferred embodiment relates to the solid oral dosage form of the invention for use in the treatment of a patient who is in need of naproxen sodium and/or for use in the treatment of pain. A more preferred embodiment relates to the solid oral dosage form of the invention for use in the treatment of arthritis pain, headache, muscular aches, toothache, backache, low back pain and/or menstrual cramps. An even more preferred embodiment relates to the solid oral dosage form of the invention for use in the treatment of low back pain. The most preferred embodiment relates to the solid oral dosage form of the invention for use in the treatment of low back pain, wherein said solid oral dosage form is a tablet comprising at least 220 mg naproxen sodium, from 90 mg to 150 mg vitamin B1, from 180 mg to 300 mg vitamin B6 and from 180 µg to 300 µg vitamin B12.

### Blend of the invention

The present invention also relates to a blend that is suitable for preparing the herein described solid oral dosage form. The blend is typically a mixture of at least two solid compounds. Preferably, the blend of the invention comprises granules and at least one source of extragranular vitamin B6, wherein said granules comprise naproxen or a pharmaceutically acceptable salt thereof and at least one binder.

In case the solid oral dosage form is a tablet, the tablet is obtainable by compressing the blend of the invention into a tablet. For compression, a Piccola "B" press equipped with 3/8" round standard concave toolings or any other tablet press can be used. In case the solid oral dosage form is a capsule, the capsule is obtainable by filling the blend of the invention into empty capsule shells such as size "00" or size "0" capsule shells. In case the solid oral dosage form is a powder, the blend of the invention is filled into containers, said containers being preferably bags, sachets or stick-packs.

One embodiment of the invention relates to a blend comprising:
a) granules, and
b) vitamin B6
wherein said granules comprise naproxen or a pharmaceutically acceptable salt thereof and at least one binder, and wherein said blend further comprises at least one pharmaceutically acceptable excipient. The person skilled in the art understands that in this embodiment, vitamin B6 is extragranular vitamin B6. Thus, alternatively, the same embodiment can be re-phrased as a blend comprising:
a) granules, and
b) extragranular vitamin B6
wherein said granules comprise naproxen or a pharmaceutically acceptable salt thereof and at least one binder, and wherein said blend further comprises at least one pharmaceutically acceptable excipient. This applies mutatis mutandis also to the following embodiments.

The choice of the pharmaceutically acceptable excipient(s) depends on the use of the blend.

In case the blend is meant to be compressed into tablets, the blend further comprises preferably at least one diluent (such as microcrystalline cellulose, dicalcium phosphate, lactose, maltodextrin, mannitol and mixtures thereof), optionally at least one disintegrant (such as croscarmellose sodium, starch, crospovidone, sodium starch glycolate and mixtures thereof) and at least one lubricant (such as magnesium stearate, polyethylene glycol (PEG), calcium stearate, talc, sodium stearyl fumarate and mixtures thereof). Thus, a preferred embodiment of the invention relates to a blend comprising:
a) granules, and
b) extragranular pyridoxine hydrochloride
wherein said granules comprise naproxen sodium and at least one binder, and wherein said blend further comprises at least one diluent (preferably microcrystalline cellulose) and at least one lubricant (preferably magnesium stearate).

In case the blend is meant to be filled into capsule shells, the blend comprises optionally at least one flowing agent such as silicon dioxide; the addition of a lubricant is optional, too. Thus, a preferred embodiment of the invention relates to a blend comprising:
a) granules, and
b) extragranular pyridoxine hydrochloride
wherein said granules comprise naproxen sodium and at least one binder, and wherein said blend does preferably not contain any lubricant.

In case the blend is meant to be filled into sachets, stick-packs or alike, the blend further comprises preferably at least one water-soluble diluent such as mannitol and/or at least one taste masking agent such as a sweetener (e.g. sucralose), an acid (e.g. citric acid or malic acid) or a flavor (e.g. lemon favor or raspberry flavor). Thus, an also preferred embodiment of the invention relates to a blend comprising:
a) granules, and
b) extragranular pyridoxine hydrochloride
wherein said granules comprise naproxen sodium and at least one binder, and wherein said blend further comprises at least one water-soluble diluent and at least one taste masking agent, said taste masking agent being preferably a combination of a sweetener, at least one edible acid and at least one pharmaceutically acceptable flavor.

Usually, the size of the granules does not depend on the use of the blend.

In one embodiment, at least 80 wt.-%, preferably at least 85 wt.-% and most preferably at least 90 wt.-% of the granules pass mesh 16, based on the total weight of the granules, and/or at least 80 wt.-%, preferably at least 85 wt.-% and most preferably at least 90 wt.-% of the granules are retained by mesh 200, based on the total weight of the granules. In an alternative embodiment, at least 80 wt.-%, preferably at least 85 wt.-% and most preferably at least 90 wt.-% of the granules pass mesh 18, based on the total weight of the granules, and/or at least 80 wt.-%, preferably at least 85 wt.-% and most preferably at least 90 wt.-% of the granules are retained by mesh 200, based on the total weight of the granules. In yet another embodiment, at least 80 wt.-%, preferably at least 85 wt.-% and most preferably at least 90 wt.-% of the granules pass mesh 16, based on the total weight of the granules, and/or at least 80 wt.-%, preferably at least 85 wt.-% and most preferably at least 90 wt.-% of the granules are retained by mesh 170, based on the total weight of the granules. In still another embodiment, at least 80 wt.-%, preferably at least 85 wt.-% and most preferably at least 90 wt.-% of the granules pass mesh 18, based on the total weight of the granules, and/or at least 80 wt.-%, preferably at least 85 wt.-% and most preferably at least 90 wt.-% of the granules are retained by mesh 170, based on the total weight of the granules.

A particle size conversion table is given below.

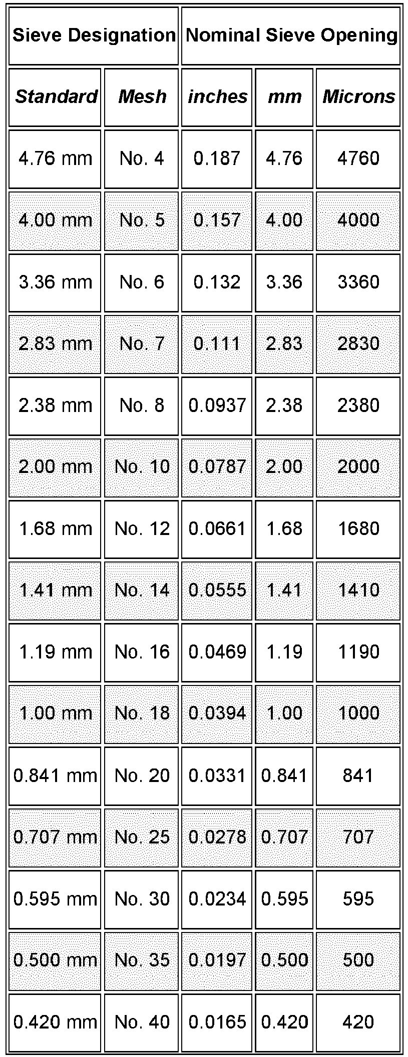

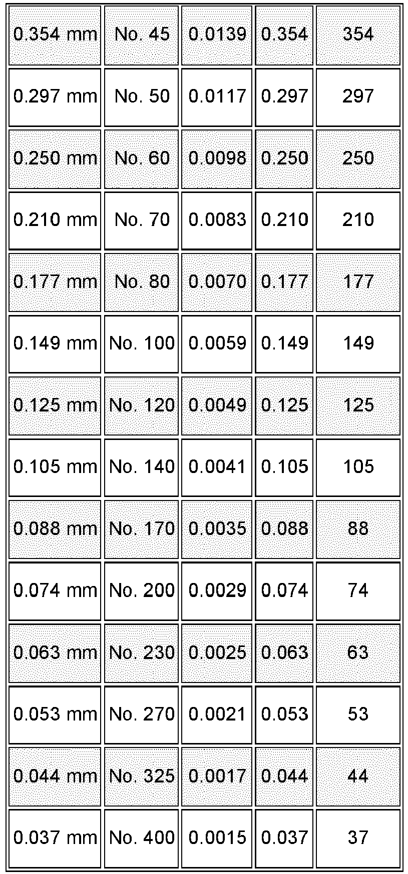

According to the method of the invention, vitamin B6 is an extragranular compound. However, although not preferred, this does not exclude the possibility that the herein described granules comprise a small amount of vitamin B6. In one embodiment, the herein described granules comprise less than 10 weight-%, preferably less than 5 weight-%, more preferably less than 1 weight-% and most preferably less than 0.1 weight-% vitamin B6, based on the total weight of the granules. A preferred embodiment of the invention relates to a blend comprising:
a) granules, and
b) extragranular vitamin B6, being preferably pyridoxine hydrochloride wherein said granules comprise naproxen sodium and at least one binder, and wherein granules comprise less than 1 weight-% and preferably less than 0.1 weight-% vitamin B6, and
   wherein said blend further comprises at least one pharmaceutically acceptable excipient.

Any binder suitable for the granulation of naproxen can be used. In case of naproxen sodium, wet-granulation with water as a processing solvent is preferred, and thus, the binder is preferably water-soluble. A particularly preferred binder is water-soluble polyvinylpyrrolidone such as PVP K30.

In a preferred embodiment, the blend of the invention comprises, in addition to vitamin B6, at least one additional B-vitamin such as vitamin B1 or vitamin B12.

If present, vitamin B1 is preferably an intragranular compound. When compressing such blend into tablets, intragranular addition of vitamin B1 increases hardness of the obtained tablet. In addition, the size of the tablet can be reduced by omitting the diluent or by reducing the amount of diluent.

If present, vitamin B12 is preferably an extragranular compound as this simplifies the granulation of naproxen sodium. Thus, one embodiment of the invention relates to a blend comprises:
a) granules,
b) extragranular vitamin B6,
c) optionally extragranular vitamin B12, and
wherein said granules comprise naproxen sodium, vitamin B1 and at least one binder. Preferably, the granules of the invention comprise from 5 weight-% to 80 weight-%, preferably from 20 weight-% to 60 weight-%, more preferably from 30 weight-% to 55 weight-% and most preferably from 35 weight-% to 50 weight-% vitamin B1, based on the total weight of the granules.

According to the preferred method of the invention, vitamin B6 is an extragranular compound whereas vitamin B1 is an intragranular compound. However, although not preferred, this does not exclude the possibility that the blend comprise a small amount of intragranular vitamin B6 and/or a small amount of extragranular vitamin B1. In one embodiment, the herein described granules comprise less than 10 weight-%, preferably less than 5 weight-%, more preferably less than 1 weight-% and most preferably less than 0.1 weight-% vitamin B6, based on the total weight of the granules. In another embodiment, the herein described blend comprise less than 10 weight-%, preferably less than 5 weight-%, more preferably less than 1 weight-% and most preferably less than 0.1 weight-% extragranular vitamin B1, based on the total weight of the blend. A preferred embodiment of the invention relates to a blend comprising:
a) granules,
b) extragranular vitamin B6,
c) optionally extragranular vitamin B12, and

wherein said granules comprise naproxen sodium, vitamin B1 and at least one binder, and
wherein granules comprise less than 1 weight-% and preferably less than 0.1 weight-% vitamin B6, based on the total weight of the granules and wherein said blend comprises less than 1 weight-% and preferably less than 0.1 weight-% extragranular vitamin B1.

A more preferred embodiment of the invention relates to a blend comprising:
a) granules, and
b) extragranular pyridoxine hydrochloride
c) extragranular vitamin B12, and

wherein said granules comprise naproxen sodium, vitamin B1 and at least one water-soluble binder, and
wherein granules comprise less than 1 weight-% and preferably less than 0.1 weight-% vitamin B6, based on the total weight of the granules,
wherein said blend comprises less than 1 weight-% and preferably less than 0.1 weight-% extragranular vitamin B1, based on the total weight of the blend and
wherein said blend further comprises at least one pharmaceutically acceptable excipient.

According to the method of the invention, vitamin B12 is an extragranular compound. However, although not preferred, this does not exclude the possibility that the herein described granules comprise a small amount of vitamin B12. In one embodiment, the herein described granules comprise less than 1 weight-%, preferably less than 0.1 weight-%, more preferably less than 0.01 weight-% and most preferably less than 0.001 weight-% vitamin B12, based on the total weight of the granules.

The addition of vitamin B12 to a fixed-dose combination is challenging. Crystalline vitamin B12 is commercially available but is very sensitive to light. Thus, unless a very extensive packaging is used, the required shelf life will not be achieved when using crystalline vitamin B12. In addition, the amount of vitamin B12 that needs to be added (based on RDA) is very small. In most cases, 200 µg or less is sufficient. Due to the small amount, it is very difficult or even impossible to reach acceptable content uniformity. Thus, when adding crystalline vitamin B12, there is a high risk that the content of vitamin B12 will vary from one tablet to the other. Extragranular addition of vitamin B12 allows using a spray-dried formulation of vitamin B12. Using a spray-dried formulation of vitamin B12 such as "vitamin B12 0.1% WS" (available at DSM^{®} Nutritional Products, Switzerland) increases content uniformity of vitamin B12 and stability. Thus, a preferred embodiment of the invention relates to a blend comprising:
a) granules,
b) extragranular pyridoxine hydrochloride, and
c) a spray-dried formulation of vitamin B12, and

wherein said granules comprise naproxen sodium, vitamin B1 and at least one water-soluble binder, and
wherein said blend comprises less than 1 weight-% and preferably less than 0.1 weight-% extragranular vitamin B1, based on the total weight of the blend, and
wherein granules comprise less than 1 weight-% and preferably less than 0.1 weight-% vitamin B6, based on the total weight of the granules, and wherein said blend further comprises at least one pharmaceutically acceptable excipient.

The most preferred solid oral dosage form of the invention is a tablet that comprises at least 220 mg naproxen sodium and that is obtainable by compressing a blend, wherein said blend comprises:
a) granules,
b) extragranular pyridoxine hydrochloride, and
c) a spray-dried formulation of vitamin B12, and

wherein said granules comprise naproxen sodium, vitamin B1 and at least one water-soluble binder, and
wherein said vitamin B1 is preferably thiamine hydrochloride, thiamine mononitrate or benfotiamine, and wherein said vitamin B1 is most preferably thiamine hydrochloride, and
wherein said blend comprises less than 1 weight-% and preferably less than 0.1 weight-% extragranular vitamin B1, based on the total weight of the blend, and
wherein granules comprise less than 1 weight-% and preferably less than 0.1 weight-% vitamin B6, based on the total weight of the granules, and
wherein said blend further comprises at least one diluent, at least one disintegrant and at least one lubricant.

### Method of preparing the solid oral dosage form of the invention

The present invention also relates to a method of preparing a solid oral dosage form which comprises naproxen or a pharmaceutically acceptable salt thereof, vitamin B6 and optionally at least one additional B-vitamin. Thus, the present invention also relates to method of preparing a fixed-dose combination (FDC) of naproxen and at least one vitamin of the vitamin B complex.

The method of the present invention relates preferably to wet-granulation, using preferably water as processing solvent. For granulation, any suitable equipment can be used such as wet granulation was conducted using KitchenAid Professional 600 mixer.

The method of the present invention comprises the extragranular addition of vitamin B6 to an intragranular composition, wherein said intragranular composition comprises naproxen or a pharmaceutically acceptable salt thereof and at least one binder. In this embodiment, the method comprises preferably the steps:
a) wet granulating with water as a processing solvent naproxen or a pharmaceutically acceptable salt thereof in the presence of at least one binder to obtain granules
b) drying the granules obtained in step a)
c) optionally milling the granules obtained in step b)
d) mixing the granules obtained in step c) with vitamin B6 and at least one pharmaceutically acceptable excipient, and
e) optionally compressing the blend obtained in step d) into tablets, or filling the blend obtained in step d) into capsules or filling the blend obtained in step d) into containers, said containers being preferably bags, sachets or stick-packs.

In a preferred embodiment, the present invention relates to a method of preparing a solid oral dosage form which comprises naproxen or a pharmaceutically acceptable salt thereof, vitamin B6, vitamin B1 and optionally at least one additional B-vitamin. In this embodiment, the method of the present invention comprises the extragranular addition of vitamin B6 to an intragranular composition, wherein said intragranular composition comprises naproxen or a pharmaceutically acceptable salt thereof, vitamin B1 and at least one binder. Thus, the method comprises preferably the steps:
a) wet granulating with water as a processing solvent naproxen or a pharmaceutically acceptable salt thereof in the presence of at least one binder and vitamin B1 to obtain granules
b) drying the granules obtained in step a)
c) optionally milling the granules obtained in step b)
d) mixing the granules obtained in step c) with vitamin B6 and at least one pharmaceutically acceptable excipient, and
e) optionally compressing the blend obtained in step d) into tablets, or filling the blend obtained in step d) into capsules or filling the blend obtained in step d) into containers, said containers being preferably bags, sachets or stick-packs.

In an also preferred embodiment, the present invention relates to method of preparing a solid oral dosage form which comprises naproxen or a pharmaceutically acceptable salt thereof, vitamin B6, vitamin B12 and optionally at least one additional B-vitamin. In this embodiment, the method of the present invention comprises the extragranular addition of vitamin B6 and vitamin B12 to an intragranular composition, wherein said intragranular composition comprises naproxen or a pharmaceutically acceptable salt thereof and at least one binder. Thus, the method comprises preferably the steps:
a) wet granulating with water as a processing solvent naproxen or a pharmaceutically acceptable salt thereof in the presence of at least one binder to obtain granules
b) drying the granules obtained in step a)
c) optionally milling the granules obtained in step b)
d) mixing the granules obtained in step c) with vitamin B6, at least one pharmaceutically acceptable excipient and at least one spray-dried formulation of vitamin B12, and
e) optionally compressing the blend obtained in step d) into tablets, or filling the blend obtained in step d) into capsules or filling the blend obtained in step d) into containers, said containers being preferably bags, sachets or stick-packs.

In a more preferred embodiment, the present invention relates to method of preparing a solid oral dosage form which comprises naproxen or a pharmaceutically acceptable salt thereof, vitamin B6, vitamin B1 and vitamin B12. In this embodiment, the method of the present invention comprises the extragranular addition of vitamin B6 and vitamin B12 to an intragranular composition, wherein said intragranular composition comprises naproxen or a pharmaceutically acceptable salt thereof, vitamin B1 and at least one binder. Thus, the method comprises preferably the steps:
a) wet granulating with water as a processing solvent naproxen or a pharmaceutically acceptable salt thereof in the presence of at least one binder and vitamin B1 to obtain granules
b) drying the granules obtained in step a)
c) optionally milling the granules obtained in step b)
d) mixing the granules obtained in step c) with vitamin B6, at least one pharmaceutically acceptable excipient and at least one spray-dried formulation of vitamin B12, and
e) optionally compressing the blend obtained in step d) into tablets, or filling the blend obtained in step d) into capsules or filling the blend obtained in step d) into containers, said containers being preferably bags, sachets or stick-packs.

The preferred naproxen is naproxen sodium. The preferred vitamin B6 is pyridoxine hydrochloride. Vitamin B1 is preferably thiamine hydrochloride, thiamine mononitrate, benfotiamine or a mixture thereof. The most preferred vitamin B1 is thiamine hydrochloride. Thus, the method of the present invention comprises more preferably the steps:
a) wet granulating with water as a processing solvent naproxen sodium in the presence of at least one binder and thiamine hydrochloride to obtain granules
b) drying the granules obtained in step a)
c) optionally milling the granules obtained in step b)
d) mixing the granules obtained in step c) with, at least one pharmaceutically acceptable excipient, pyridoxine hydrochloride and at least one spray-dried formulation of vitamin B12, and
e) optionally compressing the blend obtained in step d) into tablets, or filling the blend obtained in step d) into capsules or filling the blend obtained in step d) into containers, said containers being preferably bags, sachets or stick-packs.

### Figures

*FIGURE* 1 shows the tablets manufactured in example 9.
*FIGURE* 2 shows the drink prepared in example 11.

### Examples

### Example 1 (control; naproxen sodium only)

Example 1 is a control experiment relating to naproxen sodium only (i.e. without any vitamins). First, the physical properties of naproxen sodium (source: Xi'an Wharton Biological Technology Co., Ltd., China) were evaluated. The result of this evaluation is shown in below Table 1.

**Table 1. Physical characterizations of Naproxen Sodium**

| **Material** | **BD (g/ml)** | **TD (g/ml)** | **Carr Index** | **Description** |
|---|---|---|---|---|
| Naproxen Sodium | 0.30 | 0.55 | 45 | very lumpy |

Table 1 shows the bulk density (BD), tapped density (TD), and the Carr Index. The Carr Index is calculated by the formula 100x(1-BD/TD) and is used as an indication of the flowability. A Carr Index greater than 25 is considered to be an indication of poor flowability. As it can be seen in Table 1, naproxen sodium is a lumpy powder with poor flowability. This preliminary evaluation indicates that naproxen sodium is not suitable for direct compression.

Therefore, naproxen sodium was wet granulated using PVP (polyvinylpyrrolidone) as binder. To do so, dry naproxen sodium was put in a granulator. Then, an aqueous binder solution comprising PVP K30 (available at Ashland) and water was gradually added, and some time was allowed for the kneading of granules. The wet granular mass was then transferred to be spread on a tray. Granules were first dried at 86 °C for 2.5 hours and then dried at 50 °C overnight. Loss on drying (LOD) values of the granules were between 3-4 wt.-%, based on the total weight of the wet granules. Finally, the dried granules were milled using a Fitz Mill equipped with 4 mm screen (milling speed: 20-30 Hz; blade forward). Particles were classified until retains above 20-mesh screen were less than 30%. The composition of the thus obtained naproxen sodium granules is shown in below Table 2, indicated as mg/tablet when targeting a tablet comprising 221.11 mg naproxen sodium (corresponding to a label claim of 220 mg naproxen).

**Table 2. Formulation of granulation trial (mg/tablet)**

| **Material** | **mg/tablet** | **Label claim (mg)** | **Purity (%)** | **Overage (wt.-%)** | **mg/tablet** |
|---|---|---|---|---|---|
| Naproxen Sodium | 221.11 | 220 | 99.5 | 0 | 221.11 |
| PVP K30 | 4.42 | | | | |
| Total | 225.53 | | | | |

Purity is a nondimensional factor.

The thus obtained dried granules had a significantly higher density and much smaller Carr Index than the ungranulated naproxen sodium. The result of the applicable test is shown in below Table 3.

**Table 3. Physical properties of wet granulation trials and raw material**

| **Description** | **PVP mg/tablet** | **BD (g/ml)** | **TD (g/ml)** | **Carr Index** |
|---|---|---|---|---|
| Naproxen sodium | 0 | 0.30 | 0.55 | 45 |
| Naproxen sodium granules | 4.42 | 0.57 | 0.66 | 14 |

To produce tablets, the obtained naproxen sodium granules were blended with suitable excipients: microcrystalline cellulose (MCC) as diluent; croscarmellose sodium as disintegrant; and magnesium stearate as lubricant. The tablets were produced on a Piccola B rotary tablet press equipped with 3/8" standard concave round tooling at 25 rpm press speed. Naproxen tablets (label claim: 200 mg) were compressed with different compression force (1000 Lbs., 2000 Lbs., and 3000 Lbs.). The result of this tableting trial is shown in Table 4.

**Table 4. Tableting trial of naproxen sodium granules**

| **ingredient** | | **mg/tablet** |
|---|---|---|
| Naproxen sodium granules | Naproxen Sodium | 221.11 |
| | PVP K30 | 4.42 |
| MCC 200 | | 71.47 |
| Croscarmellose Sodium | 1.50 | |
| Magnesium Stearate | 1.50 | |
| Total (mg/tablet) | 300.00 | |
| | | |

| **Compression profile** | **Hardness (kp)** | |
|---|---|---|
| 1000 Lbs. (4448.2 N) | 4.9 | |
| 2000 Lbs. (8896.4 N) | 8.5 | |
| 3000 Lbs. (13344.6 N) | 9.0 | |

The hardness of thus obtained naproxen tablets was measured with a Sotax HT10 hardness tester by checking the breaking force and ranged from 4.9 kp to 9.0 kp (1 kp=1 kg force=9.8 N). Example 1 shows that the chosen excipients, equipment and process conditions are suitable for manufacturing a solid oral dosage form that comprises naproxen sodium.

### Example 2 (comparative example; intragranular addition of vitamin B6)

In Example 2, the tableting trial of example 1 was repeated. However, in example 2, naproxen sodium was granulated together with vitamin B6. As a source of vitamin B6, pyridoxine hydrochloride (available at DSM^{®} Nutritional Products, Switzerland) was used. The composition of the thus obtained granules is shown in below Table 5, indicated as mg/tablet.

**Table 5. Formulation of granulation trial (mg/tablet)**

| **Material** | **mg/tablet** | **Label claim (mg)** | **Purity (%)** | **Conversion factor** | **Overage (wt.-%)** | **mg/tablet** |
|---|---|---|---|---|---|---|
| Naproxen Sodium | 221.11 | 220 | 99.5 | NA | 0 | 221.11 |
| Pyridoxine Hydrochloride | 267.41 | 200 | 100 | 0.8227 | 10 | 267.41 |
| PVP K30 | 4.42 | | | | | |
| Total | 492.94 | | | | | |

Overage is the amount of dietary ingredient such as vitamins that is more than the target amount. It is indicated as wt.-%, based on the weight of the target amount. In above Table 5, 267.41 mg pyridoxine hydrochloride * 0.8227 = 220 mg pyridoxine hydrochloride has been put into each tablet. This corresponds to an overage of 10 wt.-% (200 mg + 10 wt.-% = 220 mg). Typically, overages are meant to compensate for loss due to degradation during manufacturing or storage.

The thus obtained dried granules had a significantly higher density and much smaller Carr Index than both, the ungranulated naproxen sodium and the ungranulated pyridoxine hydrochloride. The result is shown in below Table 6.

**Table 6. Physical properties of wet granulation trials and raw materials**

| **Description** | **PVP mg/tablet** | **BD (g/ml)** | **TD (g/ml)** | **Carr Index** |
|---|---|---|---|---|
| Naproxen sodium | 0 | 0.30 | 0.55 | 45 |
| Naproxen sodium granules | 4.42 | 0.57 | 0.66 | 14 |
| Pyridoxine Hydrochloride | 0 | 0.44 | 0.62 | 30 |
| Granules comprising naproxen sodium and Pyridoxine Hydrochloride | 4.42 | 0.51 | 0.65 | 22 |

The preliminary evaluation indicates that the obtained granules comprising naproxen sodium and pyridoxine hydrochloride are suitable for direct compression. Tablets were then compressed in the same manner as in Example 1. The result of this tableting trial is shown in below Table 7.

**Table 7. Tableting trial of granules comprising naproxen sodium and pyridoxine hydrochloride**

| **Material** | | **mg/tablet** |
|---|---|---|
| Granules comprising naproxen sodium and pyridoxine hydrochloride | Naproxen Sodium | 221.11 |
| | Pyridoxine Hydrochloride | 267.41 |
| | PVP K30 | 4.42 |
| MCC 200 | | 71.47 |
| Croscarmellose Sodium | | 1.50 |
| Magnesium Stearate | | 1.50 |
| Total (mg/tablet) | | 567.41 |
| | | |

| **Compression profile** | | **Hardness (kp)** |
|---|---|---|
| 1000 Lbs. | | Capping |
| 2000 Lbs. | | Capping |
| 3000 Lbs. | | Capping |

Although a Carr index of less than 25 had been measured (which indicates that the granules should be suitable for direct compression), it was surprisingly not possible to compress tablets without capping. Due to capping, hardness of the tablets could not be measured.

### Example 3 (extragranular addition of vitamin B6)

Example 2 was repeated. However, in Example 3, pyridoxine hydrochloride was not granulated together with naproxen sodium. Instead, the naproxen sodium granules of Example 1 were used and pyridoxine hydrochloride was added afterwards together with the required excipients. The result of this tableting trial is shown in below Table 8.

**Table 8. Tableting trial of granules comprising naproxen sodium only; Pyridoxine Hydrochloride was added afterwards together with the required excipients**

| **Material** | | **mg/tablet** |
|---|---|---|
| Naproxen sodium granules | Naproxen Sodium | 221.11 |
| | PVP K30 | 4.42 |
| Pyridoxine Hydrochloride | | 267.41 |
| MCC 200 | | 71.47 |
| Croscarmellose Sodium | | 1.50 |
| Magnesium Stearate | | 1.50 |
| Total (mg/tablet) | | 567.41 |
| | | |

| **Compression profile** | | **Hardness (kp)** |
|---|---|---|
| 1000 Lbs. | | 6.3 |
| 2000 Lbs. | | 9.7 |
| 3000 Lbs. | | 10.1 |

In example 3, no capping was observed although the same amounts of the same ingredients (i.e. as in example 2) were used. In addition to this surprising result, tablet hardness was slightly higher compared to the control (cf. example 1, table 4) at all tested compression forces.

### Example 4 (extragranular addition of vitamin B1)

In example 4, as fixed-dose combination of naproxen sodium and vitamin B1 was prepared. As a source of vitamin B1, thiamine hydrochloride (available at DSM^{®} Nutritional Products, Switzerland) was used. In consideration of the failure in example 2, the approach of example 3 was repeated. Thus, naproxen sodium granules of example 1 were used and thiamine hydrochloride was added afterwards together with the required excipients. The result of this tableting trial is shown in below Table 9.

**Table 9. Tableting trial of granules comprising naproxen sodium only; thiamine hydrochloride was added afterwards together with the required excipients**

| **Material** | | **mg/tablet** |
|---|---|---|
| Naproxen sodium granules | Naproxen Sodium | 221.11 |
| | PVP K30 | 4.42 |
| Thiamine Hydrochloride | | 147.18 |
| MCC 200 | | 71.47 |
| Croscarmellose Sodium | | 1.50 |
| Magnesium Stearate | | 1.50 |
| Total (mg/tablet) | | 447.18 |
| | | |

| **Compression profile** | | **Hardness (kp)** |
|---|---|---|
| 1000 Lbs. | | Blend was too fluffy to be compressed into tablet with standard tooling |
| 2000 Lbs. | | Blend was too fluffy to be compressed into tablet with standard tooling |
| 3000 Lbs. | | Blend was too fluffy to be compressed into tablet with standard tooling |

Example 4 was not successful because the blend is too fluffy to reach the target tablet weight with 3/8" standard concave round tooling. Thus, the approach of example 3 failed when replacing vitamin B6 with vitamin B1.

### Example 5 (intragranular addition of vitamin B1)

In Example 5, the tableting trial of example 2 was repeated, wherein vitamin B6 was replaced with vitamin B1. Thus, naproxen sodium was granulated together with vitamin B1. Three different sources of vitamin B1 were tested: thiamine hydrochloride, thiamine mononitrate (both available at DSM^{®} Nutritional Products, Switzerland) and benfotiamine (available at Xi'an Wharton Biological Technology Co., Ltd., China) were used. The composition of the thus obtained granules is shown in below Table 10 indicated as mg/tablet. Label claim of B1 is 100 mg. The corresponding conversion factor is based on molecular weights, potency, and moisture content from historical batches. There is 10 wt.-% overage for all B1 forms.

**Table 10. Formulations of granulation trials (mg/tablet)**

| **Material** | **Formulation 1 mg/tablet** | **Formulation 2 mg/tablet** | **Formulation 3 mg/tablet** | **Label claim (mg)** | **Purity (%)** | **Conversion factor** | **Overage (wt.-%)** | **mg/tablet** |
|---|---|---|---|---|---|---|---|---|
| Naproxen Sodium | 221.11 | 221.11 | 221.11 | 220 | 99.5 | NA | 0 | 221.11 |
| Thiamine Hydrochloride | 147.18 | / | / | 100 | 100 | 0.7474 | 10 | 147.18 |
| Thiamine Mononitrate | / | 135.70 | / | 100 | 100 | 0.8106 | 10 | 135.70 |
| Benfotiamine | / | / | 194.73 | 100 | 100 | 0.5649 | 10 | 194.73 |
| PVP K30 | 4.42 | 4.42 | 4.42 | | | | | |
| Total | 372.71 | 361.23 | 420.26 | | | | | |

The thus obtained dried granules had a significantly higher density and much smaller Carr Index than both, the ungranulated naproxen sodium and the ungranulated vitamin B1. The result is shown in below Table 11.

**Table 11. Physical properties of wet granulation trials and raw materials**

| **Description** | **PVP mg/tablet** | **BD (g/ml)** | **TD (g/ml)** | **Carr Index** |
|---|---|---|---|---|
| Naproxen sodium | 0 | 0.30 | 0.55 | 45 |
| Thiamine hydrochloride | 0 | 0.26 | 0.46 | 44 |
| Granules comprising naproxen sodium and thiamine hydrochloride | 4.42 | 0.59 | 0.69 | 14.5 |
| Granules comprising naproxen sodium and thiamine mononitrate | 4.42 | 0.54 | 0.64 | 15.6 |
| Granules comprising naproxen sodium and benfotiamine granulation | 4.42 | 0.55 | 0.69 | 20.3 |

The evaluation indicates that the obtained granules comprising naproxen sodium and vitamin B1 are suitable for direct compression, regardless which source of vitamin B1 is used. Tablets were then compressed in the same manner as in examples 1 and 2. The compositions of the three kinds of tablets is shown in below Tables 12a, 12b and 12c.

**Table 12a. Tableting trial of granules comprising naproxen sodium and thiamine hydrochloride**

| **Material** | | **mg/tablet** |
|---|---|---|
| Granules comprising naproxen sodium and thiamine hydrochloride | Naproxen Sodium | 221.11 |
| | Thiamine Hydrochloride | 147.18 |
| | PVP K30 | 4.42 |
| MCC 200 | | 71.47 |
| Croscarmellose Sodium | | 1.50 |
| Magnesium Stearate | | 1.50 |
| Total (mg/tablet) | | 447.18 |

**Table 12b. Tableting trial of granules comprising naproxen sodium and thiamine mononitrate**

| **Material** | | **mg/tablet** |
|---|---|---|
| Granules comprising naproxen sodium and Thiamine Mononitrate | Naproxen Sodium | 221.11 |
| | Thiamine Mononitrate | 135.7 |
| | PVP K30 | 4.42 |
| MCC 200 | | 71.47 |
| Croscarmellose Sodium | | 1.50 |
| Magnesium Stearate | | 1.50 |
| Total (mg/tablet) | | 435.70 |

**Table 12c. Tableting trial of granules comprising naproxen sodium and benfotiamine**

| **Material** | | **mg/tablet** |
|---|---|---|
| Granules comprising naproxen sodium and benfotiamine | Naproxen Sodium | 221.11 |
| | Benfotiamine | 194.73 |
| | PVP K30 | 4.42 |
| MCC 200 | | 71.47 |
| Croscarmellose Sodium | | 1.50 |
| Magnesium Stearate | | 1.50 |
| Total (mg/tablet) | | 494.73 |

The tableting trials were successful, irrespective which source of vitamin B1 was used. Surprisingly, compressibility is significantly improved when vitamin B1 is granulated together with naproxen sodium. This effect occurred for all tested sources of vitamin B1 but was particularly strong when using thiamine hydrochloride or benfotiamine as source of vitamin B1. At 2000 lbs., hardness of the obtained tablet increased from 8.5 kp (control; example 1, table 4) to 16 kp for thiamine hydrochloride and to 17.2 kp for benfotiamine. This is an increase of approx. 100%, compared to the control.

### Example 6 (reducing the tablet size; vitamin B1)

In Example 6, the tableting trial of example 5 was repeated. However, with the finding of B1's improving naproxen compressibility, the diluent (MCC 200) was completely removed from the tablet formulation. The most promising sources of vitamin B1 (i.e. thiamine hydrochloride and benfotiamine; cf. example 5) were used. The result of this very ambitious tableting trial is shown in below Tables 13a and 13b.

**Table 13a. Tableting trial of granules comprising naproxen sodium and thiamine hydrochloride, but no MCC 200**

| **Material** | | **mg/tablet** |
|---|---|---|
| Granules comprising naproxen sodium and thiamine hydrochloride | Naproxen Sodium | 221.11 |
| | Thiamine Hydrochloride | 147.18 |
| | PVP K30 | 4.42 |
| MCC 200 | | 0.00 |
| Croscarmellose Sodium | | 1.50 |
| Magnesium Stearate | | 1.50 |
| Total (mg/tablet) | | 375.71 |
| | | |

| **Compression profile** | | **Hardness (kp)** |
|---|---|---|
| 1000 Lbs. | | 7.1 |
| 2000 Lbs. | | 11.8 |
| 3000 Lbs. | | 12.5 |

**Table 13b. Tableting trial of granules comprising naproxen sodium and benfotiamine, but no MCC 200**

| **Material** | | **mg/tablet** |
|---|---|---|
| Granules comprising naproxen sodium and benfotiamine | Naproxen Sodium | 221.11 |
| | Benfotiamine | 194.73 |
| | PVP K30 | 4.42 |
| MCC 200 | | 0.00 |
| Croscarmellose Sodium | | 1.50 |
| Magnesium Stearate | | 1.50 |
| Total (mg/tablet) | | 423.26 |
| | | |

| **Compression profile** | | **Hardness (kp)** |
|---|---|---|
| 1000 Lbs. | | 8.3 |
| 2000 Lbs. | | 13.3 |
| 3000 Lbs. | | 15.7 |

The tableting trials in example 6 were successful, regardless whether thiamine hydrochloride or benfotiamine was used as source of vitamin B1. Despite of the lack of diluent, the obtained tablets had still a higher hardness than the control (cf. example 1, table 4). Thus, example 6 shows that it is possible to reduce the amount of diluent dramatically when granulating naproxen sodium together with vitamin B1. A reduction of the amount of diluent is very meaningful for a fixed-dose combination that comprises a relatively large amount of various active pharmaceutically ingredients (APIs). In case of thiamine hydrochloride as source of vitamin B1, the tablet weight could be reduced from 447.18 mg to 375.71 mg without changing the label claim. This corresponds to a tablet weight reduction of 16%. In case of benfotiamine as source of vitamin B1, the reduction of tablet weight was smaller but still meaningful (reduction 14.4% from 494.73 mg to 423.26 mg without changing the label claim). Therefore, in the context of the present invention, thiamine hydrochloride is the preferred source of vitamin B1.

### Example 7 (intragranular addition of vitamin B12)

Vitamin B12 crystals are commercially available at DSM^{®} Nutritional Products, Switzerland. DSM^{®} also offers spray-dried forms of vitamin B12. In example 7, a spray-dried formulation of vitamin B12 was used (i) to increase shelf life of the fixed-dose combination and (ii) to improve content uniformity.

In example 7, the tableting trial of example 2 was repeated. However, in example 7, vitamin B6 was replaced with a spray-dried formulation of vitamin B12. Thus, naproxen sodium was granulated together with the spray-dried formulation of vitamin B12.

Example 7 failed because the spray-dried formulation of vitamin melted at the drying step, i.e. the granules formed a useless paste at the drying step. Thus, the tested spray-dried formulation of vitamin B12 is not suitable for wet granulation because wet granulation involves a drying step.

### Example 8 (extragranular addition of vitamin B12)

In example 8, as fixed-dose combination of naproxen sodium and vitamin B12 was prepared. As a source of vitamin B12, a spray-dried formulation of vitamin B12 was used (available at DSM^{®} Nutritional Products under the tradename vitamin B12 0.1 % WS). In consideration of the failure in example 7, the approach of example 3 was repeated. Thus, naproxen sodium granules of example 1 were used and the spray-dried formulation of vitamin B12 was added afterwards together with the required excipients. The result of this tableting trial is shown in below Table 14.

**Table 14. Tableting trial of granules comprising naproxen sodium only; source of vitamin B12 was added afterwards together with the required excipients**

| **Material** | | **mg/tablet** |
|---|---|---|
| Naproxen sodium granules | Naproxen Sodium | 221.11 |
| | PVP K30 | 4.42 |
| Spray-dried formulation of vitamin B12 | | 50 |
| MCC 200 | | 71.47 |
| Croscarmellose Sodium | | 1.50 |
| Magnesium Stearate | | 1.50 |
| Total (mg/tablet) | | 350.00 |
| | | |

| **Compression profile** | | **Hardness (kp)** |
|---|---|---|
| 1000 Lbs. | | 4.3 |
| 2000 Lbs. | | 8.0 |
| 3000 Lbs. | | 11.2 |

Example 8 was successful. Reasonably hard tablets were obtained, regardless whether a compression force of 1000 lbs., 2000 lbs., or 3000 lbs. was applied.

### Example 9 (tablet with breaking notch)

In example 9, a tablet comprising naproxen and three different B-vitamins was prepared. First, granules comprising naproxen sodium and vitamin B1 were prepared, similar to example 5 (cf. table 10). The thus obtained granules were then blended with vitamin B6, a spray-dried formulation of vitamin B12 and the required excipients. The blend was then compressed into tablets having a target tablet weight of 1100 mg. The composition of the thus obtained tablets is shown in below Table 15.

**Table 15. Tablet comprising naproxen (200 mg), vitamin B1 (100 mg), vitamin B6 (200 mg) and vitamin B12 (200 µg)**

| | | | **Concentration** | | |
|---|---|---|---|---|---|
| **Material** | **Claim (mg)** | **Conversion factor** | **(wt.-%, based on total weight of compound)** | **Overage (wt.-%)** | **mg/tablet** |
| Naproxen & vitamin B1 granules | | | | | 372.71 |
| Pyridoxine Hydrochloride | 200 | 0.8227 | | 10 | 267.41 |
| Vitamin B12 0.1% WS | 0.2 | | 0.001 | 30 | 260.00 |
| MCC 200 | | | | | 186.38 |
| Croscarmellose Sodium | | | | | 5.00 |
| Silicon Dioxide | | | | | 3.00 |
| Magnesium Stearate | | | | | 5.50 |
| Total | | | | | 1100.00 |

The tablets were produced on a Piccola "B" Press equipped with 0.3543x0.7480" oval shaped tooling with DSM^{®} logo. Compression force was 4000 lbs. Tablet hardness was 12.9 kp and friability was 0.49 %.

Friability was measured by putting minimum 10 tablets and minimum 6.5 g of tablets into a Sotax F1 friabilator and then tumbling the tablets for 100 revolutions. The weight loss/the initial weight x100% is friability. This is in accordance with USP chapter 1216, Year 2017.

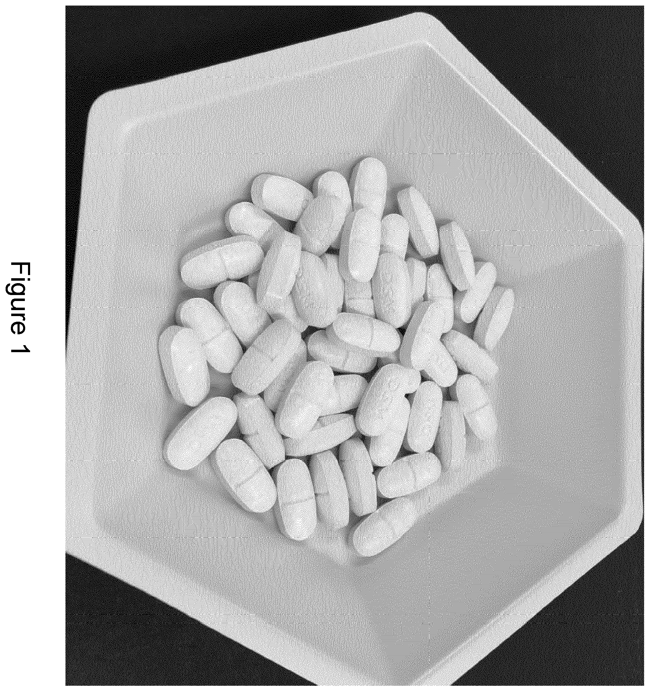

Disintegration was measured on a Sotax DT2 disintegration apparatus (purified water, in accordance with USP chapter 701, Year 2017). Disintegration time is as short as 1 min 50 sec. Individual tablet weight variation (RSD) is 1.57% (calculated based on 10 tablets). Image of the tablets of example 9 is shown in Figure 1.

### Example 10 (capsule)

In example 10, hard-shell capsules comprising naproxen and three different B-vitamins were prepared. First, granules comprising naproxen sodium and vitamin B1 were prepared, similar to example 5. The thus obtained granules were then blended with vitamin B6, a spray-dried formulation of vitamin B12 and the required excipients. The blend was then filled into capsules. The composition of the thus obtained capsules is shown in below Table 16.

**Table 16. Capsule comprising naproxen (200 mg), vitamin B1 (100 mg), vitamin B6 (200 mg) and vitamin B12 (200 µg)**

| **Material** | **Claim (mg)** | **Conversion factor** | **Concentration (wt.-%, based on total weight of compound)** | **Overage (wt.-%)** | **mg/capsule** |
|---|---|---|---|---|---|
| Naproxen & vit B1 granules | | | | | 372.71 |
| Pyridoxine Hydrochloride | 200 | 0.8227 | | 10 | 267.41 |
| Vitamin B12 0.1% WS | 0.2 | | 0.001 | 30 | 260.00 |
| MCC 200 | | | | | 6.88 |
| Silicon Dioxide | | | | | 3.00 |
| Total | | | | | 910.00 |

In contrast to the tablet formulation of example 9, the amount of MCC 200 is significantly reduced because it is a hard-shell capsule formulation instead of a tablet. Croscarmellose sodium and magnesium stearate were completely omitted. The serving size is 910 mg powder in one or more capsules. The fill weight range of the blend in size "0" capsule shells is 335.3 mg to 456.8 mg. The fill weight range of the blend in size "00" capsule shells is 445.6 mg to 615.3 mg. Therefore, the serving size of capsules are 2 capsules in either "0" or "00" capsule shells.

### Example 11 (RTM stick-pack)

In example 11, RTM (ready-to-mix) stick-packs comprising naproxen and three different B-vitamins were prepared. First, granules comprising naproxen sodium and vitamin B1 were prepared, similar to example 5. The thus obtained granules were then blended with vitamin B6, a spray-dried formulation of vitamin B12 and other suitable excipients. The blend was then filled into stick packs. The composition of the blend in the thus obtained stick-packs is shown in below Table 17.

**Table 17. RTM comprising naproxen (200 mg), vitamin B1 (100 mg), vitamin B6 (200 mg) and vitamin B12 (200 µg)**

| **Material** | **Claim (mg)** | **Conversion factor** | **Concentration (wt.-%, based on total weight of compound)** | **Overage (wt.-%)** | **mg/RTM** |
|---|---|---|---|---|---|
| Naproxen & vit B1 granules | | | | | 372.71 |
| Pyridoxine Hydrochloride | 200 | 0.8227 | | 10 | 267.41 |
| Vitamin B12 0.1% WS | 0.2 | | 0.001 | 30 | 260.00 |
| Nat Lemon Flavor | | | | | 44.81 |
| Nat Raspberry Flavor | | | | | 56.02 |
| Citric Acid | | | | | 44.81 |
| Malic Acid | | | | | 33.61 |
| Sucralose | | | | | 22.41 |
| Mannitol | | | | | 898.22 |
| Total | | | | | 2000.00 |

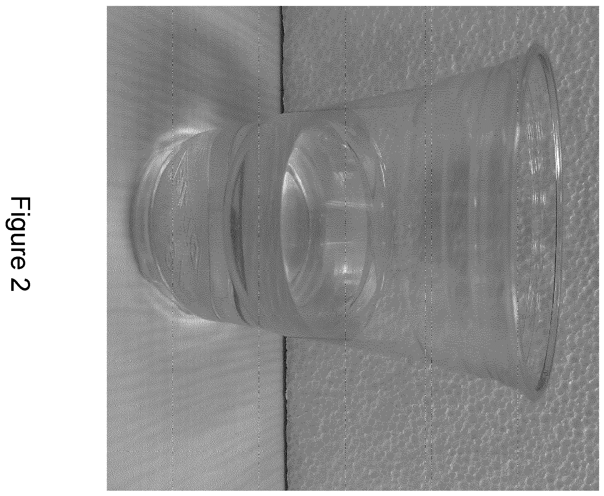

Besides the actives, flavors and acids have been added to improve taste; mannitol is used as a diluent. The serving size is 2 g/stick-pack to be dispersed in 8 oz of water. An image of the drink can be seen in Figure 2. The pink color of the drink matches the flavor.

## Claims

1. Blend comprising:
a) granules, and
b) vitamin B6
wherein said granules comprise naproxen or a pharmaceutically acceptable salt thereof and at least one binder.

2. Blend according to claim 1, wherein said blend comprises:
a) granules,
b) vitamin B6,
c) vitamin B12, and
wherein said granules comprise naproxen sodium, vitamin B1 and at least one binder, and wherein said vitamin B12 is preferably a spray-dried formulation of vitamin B12.

3. Blend according to claim 1 or 2, wherein said blend further comprises at least one pharmaceutically acceptable excipient, and wherein said blend further comprises preferably at least one diluent, at least one disintegrant, at least one lubricant, at least one flowing agent and/or at least one taste masking agent, and wherein more preferably said blend further comprises microcrystalline cellulose, mannitol, croscarmellose sodium and/or magnesium stearate.

4. Blend according to any one of claims 1 to 3, wherein at least 80% of the granules pass mesh 16, and/or wherein at least 80% of the granules are retained by mesh 200, based on the total weight of the granules comprised in the blend.

5. Blend according to any one of claims 1 to 4, wherein said granules comprise or consist of an alkali salt of naproxen and at least one water-soluble binder, and wherein said granules comprise preferably naproxen sodium and/or polyvinylpyrrolidone.

6. Blend according to any one of claims 1 to 5, wherein said granules comprise less than 10 weight-%, preferably less than 5 weight-%, more preferably less than 1 weight-% and most preferably less than 0.1 weight-% vitamin B6, based on the total weight of the granules.

7. Blend according to any one of claims 1 to 6, wherein said vitamin B6 is pyridoxine or a pharmaceutically acceptable salt thereof, and wherein said vitamin B6 is preferably pyridoxine hydrochloride.

8. Solid oral dosage form comprising the blend according to any one of claims 1 to 7.

9. Solid oral dosage form according to claim 8, wherein said solid oral dosage form is a tablet, and wherein said tablet is preferably obtained by compressing the blend according to any one of claims 1 to 7.

10. Solid oral dosage form according to claim 8, wherein said solid oral dosage form is a capsule or a powder, and wherein said powder is preferably a water-soluble or a water-dispersible powder, and wherein said powder is preferably a water-soluble powder comprising at least one taste masking agent.

11. Solid oral dosage form according to any one of claims 8 to 10 for use in the treatment of low back pain.

12. Method of preparing a solid oral dosage form,
said method comprising the extragranular addition of vitamin B6 to an intragranular composition, wherein said intragranular composition comprises naproxen or a pharmaceutically acceptable salt thereof and at least one binder.

13. Method according to claim 12, said method comprising the steps:
a) wet granulating with water as a processing solvent naproxen or a pharmaceutically acceptable salt thereof in the presence of at least one binder to obtain granules
b) drying the granules obtained in step a)
c) milling the granules obtained in step b)
d) mixing the granules obtained in step c) with vitamin B6 and at least one pharmaceutically acceptable excipient.

14. Method according to claim 13, said method comprising the steps:
a) wet granulating with water as a processing solvent naproxen or a pharmaceutically acceptable salt thereof in the presence of at least one binder and vitamin B1 to obtain granules
b) drying the granules obtained in step a)
c) milling the granules obtained in step b)
d) mixing the granules obtained in step c) with vitamin B6, at least one pharmaceutically acceptable excipient and optionally at least one spray-dried formulation of vitamin B12.

15. Method according to claim 13 or 14, said method further comprising the step:
e) compressing the blend obtained in step d) to get tablets, or filling the blend obtained in step d) into capsules or filling the blend obtained in step d) into containers, said containers being preferably bags, sachets or stick-packs.

## Patentansprüche

1. Mischung, umfassend:
a) Granulat und
b) Vitamin B6,
wobei das Granulat Naproxen oder ein pharmazeutisch unbedenkliches Salz davon und wenigstens ein Bindemittel umfasst.

2. Mischung nach Anspruch 1, wobei die Mischung Folgendes umfasst:
a) Granulat,
b) Vitamin B6,
c) Vitamin B12, und
wobei das Granulat Naproxen-Natrium, Vitamin B1 und wenigstens ein Bindemittel umfasst und wobei es sich bei dem Vitamin B12 bevorzugt um eine sprühgetrocknete Formulierung von Vitamin B12 handelt.

3. Mischung nach Anspruch 1 oder 2, wobei die Mischung ferner wenigstens einen pharmazeutisch unbedenklichen Exzipienten umfasst und wobei die Mischung ferner bevorzugt wenigstens ein Verdünnungsmittel, wenigstens ein Sprengmittel, wenigstens ein Gleitmittel, wenigstens ein Fließmittel und/oder wenigstens einen Geschmacksmaskierer umfasst und wobei bevorzugter die Mischung ferner mikrokristalline Cellulose, Mannit, Croscarmellose-Natrium und/oder Magnesiumstearat umfasst.

4. Mischung nach einem der Ansprüche 1 bis 3, wobei wenigstens 80% des Granulats Maschenweite 16 passieren und/oder wobei wenigstens 80% des Granulats durch Maschenweite 200 zurückgehalten werden, bezogen auf das Gesamtgewicht des in der Mischung enthaltenen Granulats.

5. Mischung nach einem der Ansprüche 1 bis 4, wobei das Granulat ein Alkalisalz von Naproxen und wenigstens ein wasserlösliches Bindemittel umfasst bzw. daraus besteht und wobei das Granulat bevorzugt Naproxen-Natrium und/oder Polyvinylpyrrolidon umfasst.

6. Mischung nach einem der Ansprüche 1 bis 5, wobei das Granulat kleiner 10 Gew.-%, bevorzugt kleiner 5 Gew.-%, bevorzugter kleiner 1 Gew.-% und am meisten bevorzugt kleiner 0,1 Gew.-% Vitamin B6, bezogen auf das Gesamtgewicht des Granulats, umfasst.

7. Mischung nach einem der Ansprüche 1 bis 6, wobei es sich bei dem Vitamin B6 um Pyridoxin oder ein pharmazeutisch unbedenkliches Salz davon handelt und wobei es sich bei dem Vitamin B6 bevorzugt um Pyridoxin-Hydrochlorid handelt.

8. Feste orale Darreichungsform, umfassend die Mischung nach einem der Ansprüche 1 bis 7.

9. Feste orale Darreichungsform nach Anspruch 8, wobei es sich bei der festen oralen Darreichungsform um eine Tablette handelt und wobei die Tablette bevorzugt durch Verpressen der Mischung nach einem der Ansprüche 1 bis 7 erhalten wird.

10. Feste orale Darreichungsform nach Anspruch 8, wobei es sich bei der festen oralen Darreichungsform um eine Kapsel oder ein Pulver handelt und wobei es sich bei dem Pulver bevorzugt um ein wasserlösliches oder ein wasserdispergierbares Pulver handelt und wobei es sich bei dem Pulver bevorzugt um ein wasserlösliches Pulver handelt, das wenigstens einen Geschmacksmaskierer umfasst.

11. Feste orale Darreichungsform nach einem der Ansprüche 8 bis 10 zur Verwendung bei der Behandlung von Kreuzschmerzen.

12. Verfahren zur Herstellung einer festen oralen Darreichungsform,
wobei das Verfahren die extragranuläre Zugabe von Vitamin B6 zu einer intragranulären Zusammensetzung umfasst, wobei die intragranuläre Zusammensetzung Naproxen oder ein pharmazeutisch unbedenkliches Salz davon und wenigstens ein Bindemittel umfasst.

13. Verfahren nach Anspruch 12, wobei das Verfahren die folgenden Schritte umfasst:
a) Nassgranulieren von Naproxen oder einem pharmazeutisch unbedenklichen Salz davon in Gegenwart wenigstens eines Bindemittels mit Wasser als Verarbeitungslösungsmittel unter Erhalt von Granulat,
b) Trocknen des in Schritt a) erhaltenen Granulats,
c) Mahlen des in Schritt b) erhaltenen Granulats,
d) Mischen des in Schritt c) erhaltenen Granulats mit Vitamin B6 und wenigstens einem pharmazeutisch unbedenklichen Exzipienten.

14. Verfahren nach Anspruch 13, wobei das Verfahren die folgenden Schritte umfasst:
a) Nassgranulieren von Naproxen oder einem pharmazeutisch unbedenklichen Salz davon in Gegenwart wenigstens eines Bindemittels und von Vitamin B1 mit Wasser als Verarbeitungslösungsmittel unter Erhalt von Granulat,
b) Trocknen des in Schritt a) erhaltenen Granulats,
c) Mahlen des in Schritt b) erhaltenen Granulats,
d) Mischen des in Schritt c) erhaltenen Granulats mit Vitamin B6, wenigstens einem pharmazeutisch unbedenklichen Exzipienten und gegebenenfalls wenigstens einer sprühgetrockneten Formulierung von Vitamin B12.

15. Verfahren nach Anspruch 13 oder 14, wobei das Verfahren ferner den folgenden Schritt umfasst:
e) Verpressen der in Schritt d) erhaltenen Mischung zum Erhalten von Tabletten oder Füllen der in Schritt d) erhaltenen Mischung in Behälter, wobei es sich bei den Behältern bevorzugt um Beutel, Tütchen oder Stick-Packs handelt.

## Revendications

1. Mélange comprenant :
a) des granulés, et
b) de la vitamine B6
lesdits granulés comprenant du naproxène ou un sel pharmaceutiquement acceptable correspondant et au moins un liant.

2. Mélange selon la revendication 1, ledit mélange comprenant :
a) des granulés,
b) de la vitamine B6,
c) de la vitamine B12, et
lesdits granulés comprenant du naproxène sodique, de la vitamine B1 et au moins un liant, et ladite vitamine B12 étant préférablement une formulation séchée par pulvérisation de vitamine B12.

3. Mélange selon la revendication 1 ou 2, ledit mélange comprenant en outre au moins un excipient pharmaceutiquement acceptable, et ledit mélange comprenant en outre préférablement au moins un diluant, au moins un désintégrant, au moins un lubrifiant, au moins un agent d'écoulement et/ou au moins un agent de masquage de goût, et plus préférablement ledit mélange comprenant en outre de la cellulose microcristalline, du mannitol, de la croscarmellose sodique et/ou du stéarate de magnésium.

4. Mélange selon l'une quelconque des revendications 1 à 3, au moins 80 % des granulés passant une maille 16, et/ou au moins 80 % des granulés étant retenus par une maille 200, sur la base du poids total des granulés compris dans le mélange.

5. Mélange selon l'une quelconque des revendications 1 à 4, lesdits granulés comprenant ou étant constitués d'un sel de métal alcalin de naproxène et d'au moins un liant soluble dans l'eau, et lesdits granulés comprenant préférablement du naproxène sodique et/ou de la polyvinylpyrrolidone.

6. Mélange selon l'une quelconque des revendications 1 à 5, lesdits granulés comprenant moins de 10 % en poids, préférablement moins de 5 % en poids, plus préférablement moins de 1 % en poids et le plus préférablement moins de 0,1 % en poids de vitamine B6, sur la base du poids total des granulés.

7. Mélange selon l'une quelconque des revendications 1 à 6, ladite vitamine B6 étant la pyridoxine ou un sel pharmaceutiquement acceptable correspondant, et ladite vitamine B6 étant préférablement le chlorhydrate de pyridoxine.

8. Forme posologique orale solide comprenant le mélange selon l'une quelconque des revendications 1 à 7.

9. Forme posologique orale solide selon la revendication 8, ladite forme posologique orale solide étant un comprimé, et ledit comprimé étant préférablement obtenu par compression du mélange selon l'une quelconque des revendications 1 à 7.

10. Forme posologique orale solide selon la revendication 8, ladite forme posologique orale solide étant une capsule ou une poudre, et ladite poudre étant préférablement une poudre soluble dans l'eau ou une poudre dispersable dans l'eau, et ladite poudre étant préférablement une poudre soluble dans l'eau comprenant au moins un agent de masquage de goût.

11. Forme posologique orale solide selon l'une quelconque des revendications 8 à 10 pour une utilisation dans le traitement d'une douleur du bas du dos.

12. Procédé de préparation d'une forme posologique orale solide,
ledit procédé comprenant l'ajout extragranulaire de vitamine B6 à une composition intragranulaire, ladite composition intragranulaire comprenant du naproxène ou un sel pharmaceutiquement acceptable correspondant et au moins un liant.

13. Procédé selon la revendication 12, ledit procédé comprenant les étapes :
a) granulation humide, avec de l'eau en tant qu'un solvant de traitement, de naproxène ou d'un sel pharmaceutiquement acceptable correspondant en la présence d'au moins un liant pour obtenir des granulés
b) séchage des granulés obtenus dans l'étape a)
c) broyage des granulés obtenus dans l'étape b)
d) mélange des granulés obtenus dans l'étape c) avec de la vitamine B6 et au moins un excipient pharmaceutiquement acceptable.

14. Procédé selon la revendication 13, ledit procédé comprenant les étapes :
a) granulation humide, avec de l'eau en tant qu'un solvant de traitement, de naproxène ou d'un sel pharmaceutiquement acceptable correspondant en la présence d'au moins un liant et de vitamine B1 pour obtenir des granulés
b) séchage des granulés obtenus dans l'étape a)
c) broyage des granulés obtenus dans l'étape b)
d) mélange des granulés obtenus dans l'étape c) avec de la vitamine B6, au moins un excipient pharmaceutiquement acceptable et éventuellement au moins une formulation séchée par pulvérisation de vitamine B12.

15. Procédé selon la revendication 13 ou 14, ledit procédé comprenant en outre l'étape :
e) compression du mélange obtenu dans l'étape d) pour obtenir des comprimés, ou remplissage de capsules avec le mélange obtenu dans l'étape d) ou remplissage de récipients avec le mélange obtenu dans l'étape d), lesdits récipients étant préférablement des sacs, des sachets ou des sachets-doses.
